(19) Europäisches Patentamt
European Patent Office
Office européen des brevets

(11) Numéro de publication : **0 504 236 B1**

(12) # FASCICULE DE BREVET EUROPEEN

(45) Date de publication du fascicule du brevet : **23.03.94 Bulletin 94/12**

(21) Numéro de dépôt : **91900845.8**

(22) Date de dépôt : **06.12.90**

(86) Numéro de dépôt international : **PCT/FR90/00886**

(87) Numéro de publication internationale : **WO 91/07946 13.06.91 Gazette 91/13**

(51) Int. Cl.$^5$ : **A61K 7/48,** A61K 7/06, A61K 35/80, A23L 3/3472, A23L 3/3481, A01N 31/16

(54) **UTILISATION D'EXTRAITS D'ALGUES POUR LA PREPARATION DE COMPOSITIONS PHARMACEUTIQUES, COSMETIQUES, ALIMENTAIRES OU A USAGE AGRICOLE.**

Le dossier contient des informations techniques présentées postérieurement au dépôt de la demande et ne figurant pas dans le présent fascicule.

(30) Priorité : **06.12.89 FR 8916138**

(43) Date de publication de la demande : **23.09.92 Bulletin 92/39**

(45) Mention de la délivrance du brevet : **23.03.94 Bulletin 94/12**

(84) Etats contractants désignés : **AT BE CH DE DK ES FR GB GR IT LI LU NL SE**

(56) Documents cités :
**EP-A- 0 271 133**
**WO-A-84/02652**
**DE-A- 2 651 617**
**FR-A- 2 563 414**
**Manufacturing Chemist, vol. 57, no. 5, mai 1986 (London, GB), "Natural products with antibacterial activity 6 surface active properties on the increase"page 30**
**Patent Abstracts of Japan, vol. 9, no. 125 (C-283)(1848), 30 mai 1985, & JP-A-6013709 (Nippon Carbide Kogyo K.K.) 24 janvier 1985**
**Patent Abstracts of Japan, vol. 12, no. 433 (C-543)(3280), 15 November 1988, & JP-A-63160567 (Shiraimatsu Shinyaku K.K.) 4 juillet 1988**
**W. Kern et al.: "Hagers Handbuch der pharmazeutischen Paxis", 4 ed., vol. IV Chemikalien und Drogen (Ci-G), 1973, Springer, (Berlin, DE), page 1065**

(56) Documents cités :
**S.T.N. Servers de bases de données, (Karlsruhe, DE), AN no. CA88(9):60081d, K.W.Glombitza et al.: "Antibiotics from algae. Part 21. Phlorotannin precursors in dictyota dichotoma"**
**S.T.N. Servers de bases de données, (Karlsruhe, DE), AN no. 90:265019, U. Woelwer-Rieck et al.: "Alkaline cleavage of polymeric phenols from sargassum-muticum and pelvetia-canaliculata**

(73) Titulaire : **SOCIETE D'ENGRAIS COMPOSES MINERAUX ET AMENDEMENTS (S.E.C.M.A.)**
**Zone Industrielle**
**F-22260 Pontrieux (FR)**

(72) Inventeur : **BRIAND, Xavier**
**Pors Gwen Kermouster**
**F-22740 Lézardrieux (FR)**

(74) Mandataire : **Hubert, Philippe**
**Cabinet Beau de Loménie 158, rue de l'Université**
**F-75340 Paris Cédex 07 (FR)**

EP 0 504 236 B1

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

## Description

La présente invention a pour objet une nouvelle utilisation des extraits d'algues et trouve notamment application dans la préparation de compositions pharmaceutiques, cosmétiques, alimentaires ou à usage agricole, à activité antiradicalaire.

On sait que les extraits d'algues, en raison de leurs propriétés variées, ont été proposés dans de nombreuses applications pharmaceutiques, cosmétiques, alimentaires ou agricoles.

On sait également, par le document EP-A-0271133 que certaines algues microscopiques présentent des propriétés antiradicalaires vis-à-vis du radical hydroxyle.

La présente invention est basée sur la découverte inattendue que des extraits de certaines algues macroscopiques présentent une activité antiradicalaire vis-à-vis du radical superoxide. On sait que les ions superoxide produits au cours de réactions d'oxydation sous l'effet d'oxygène moléculaire sont très actifs et attaquent notamment les protéines et les acides nucléiques.

Par conséquent, l'invention présente un intérêt remarquable notamment pour la protection des cellules de la peau.

L'invention trouve également application pour la préparation de compositions alimentaires, en raison du pouvoir protecteur des extraits d'algues vis-à vis des acides gras polyinsaturés.

Les algues utilisées conformément à la présente invention sont des algues macroscopiques vertes (Chlorophycées), brunes (Pheophycées) et rouges (Rhodophycées).

Parmi les algues brunes, l'invention s'applique notamment aux variétés des genres Fucus, Pelvetia, Ascophyllum, Himanthalia, Laminaria, Sargassum.

Parmi les algues rouges, l'invention s'applique notamment aux variétés des genres Chondrus, Mastocarpus ou Girgatina, Palmaria, Porphyra, Ceramium et Gracilaria.

Parmi les algues vertes, l'invention s'applique notamment aux variétés des genres Ulva, Enteromorpha et Codium.

Il a été découvert que toutes ces algues n'ont pas le même degré d'activité antiradicalaire.

D'une façon générale, les algues brunes présentent l'activité antiradicalaire la plus importante, et parmi ces algues, il semble que le Fucus en particulier Fucus vesiculosus soit actuellement le plus intéressant.

Parmi les autres variétés d'algues susceptibles d'être utilisées, conformément à l'invention, on peut citer Ascophyllum nodosum, Pelvetia canaliculata, Enteromorpha, Palmaria palmata, Sargassum muticum, Ceramium rubrum, Gracilaria verrucosa, Ulva lactuca, Laminaria digitata, Codium.

La préparation des extraits d'algues utilisés conformément à l'invention peut être effectuée par des procédés classiques d'extraction en phase liquide, notamment extraction aqueuse à pH contrôlé et extraction en solvant polaire, éventuellement combinés à des procédés de concentration par séchage sous vide ou par osmose inverse ou de concentration et purification par chromographie ou par ultrafiltration.

Il a également été découvert que certaines substances isolées des extraits d'algues précités, ou obtenues par synthèse chimique, en particulier les substances choisies parmi les fucols, polyfucols, diphlorétols, polyphlorétols, bifuhalols, polyfuhalols et phlorétols, présentaient une activité antiradicalaire vis-à-vis du radical superoxyde.

Par conséquent, dans la présente description, le terme "extraits d'algues" utilisé généralement, couvre également les substances actives isolées de ces extraits, ou obtenues par synthèse chimique.

La méthode de mesure du pouvoir antiradicalaire est celle de Winterbourn (J. Lab. Clin., Med. 85.337).

L'invention sera ilustrée par les exemples suivants:

### A. Exemples de préparation d'extraits d'algues conformes à la présente invention:

#### Exemple 1

On incorpore 100 g de Fucus vésiculosus dans 200 ml d'eau.

On effectue un broyage à l'aide d'un broyeur type ultra turrax pendant 10 min à température ambiante.

L'extraction est effectuée sous légère agitation pendant 24 h à température ambiante, puis la solution obtenue est filtrée.

On obtient un filtrat qui se présente sous forme de poudre.

#### Exemple 2

On incorpore 100 g de Ascophyllum nodosum dans 200 ml d'eau.

Le broyage est effectué dans les mêmes conditions qu'à l'exemple 1.

L'extraction est réalisée sous légère agitation pendant 12 h à 50°C, puis la solution est filtrée.

Exemple 3

On incorpore 100 g de Pelvetia canaliculata dans un mélange hydroalcoolique constitué par 180 ml d'eau et 20 ml d'alcool éthylique.

Le broyage est effectué dans les mêmes conditions que celles décrites à l'exemple 1.

L'extraction est réalisée sous légère agitation pendant 12 h à 50°C, puis la solution obtenue est filtrée.

Exemple 4

On incorpore 100 g de Fucus vesiculosus dans 200 ml d'eau.

Un broyage et une extraction sont réalisées dans les conditions définies à l'exemple 1, puis la solution est filtrée.

Sur le filtrat, on effectue une évaporation sous vide à température ambiante pour obtenir un extrait parfaitement sec.

Exemple 5

On incorpore 100 g d'Enteromorpha dans un mélange hydroalcoolique comportant 180 ml d'eau et 20 ml d'alcool éthylique.

Un broyage est effectué dans les conditions définies à l'exemple 1, puis on réalise une extraction sous légère agitation pendant 8 h à 40°C, puis la solution est filtrée.

Exemple 6

On incorpore 100 g de Palmaria palmata dans un mélange hydroalcoolique constitué de 180 ml d'eau et 20 ml d'alcool isopropylique.

Un broyage est effectué dans les conditions de l'exemple 1, puis on effectue une extraction sous légère agitation pendant 24 h à 30°C et la solution est filtrée.

Exemple 7

On incorpore 100 g de Sargassum muticum dans un mélange hydroalcoolique constitué de 100 ml d'eau et 100 ml de propylèneglycol.

Un broyage est effectué dans les conditions de l'exemple 1, puis on réalise une extraction sous légère agitation pendant 24 h à 20°C, et la solution obtenue est filtrée.

Exemple 8

On incorpore 100 g de Ceramium rubrum dans un mélange hydroalcoolique constitué de 190 ml d'eau et 10 ml d'alcool éthylique.

Un broyage est réalisé dans les conditions définies à l'exemple 1, puis on effectue une extraction sous légère agitation pendant 24 h à 20°C et la solution ainsi obtenue est filtrée.

Exemple 9

On incorpore 100 g de Gracilaria verrucosa dans un mélange constitué de 180 ml d'eau et 20 ml de propylèneglycol.

Un broyage est effectué dans les conditions définies à l'exemple 1, puis on réalise une extraction sous légère agitation pendant 12 h à 40°C, et la solution ainsi obtenue est filtrée.

Exemple 10

On incorpore 100 g de Ulva lactuca dans un mélange hydroalcoolique comportant 160 ml d'eau et 40 ml d'alcool éthylique.

Un broyage est réalisé dans les conditions définies à l'exemple 1, puis on effectue une extraction sous légère agitation pendant 8 h à 50°C et on filtre la solution obtenue.

Exemple 11

On incorpore 100 g de Laminaria digitata dans un mélange hydroalcoolique constitué de 190 ml d'eau et 10 ml d'alcool éthylique.

Un broyage est effectué dans les conditions définies à l'exemple 1, puis on réalise une extraction sous légère agitation pendant 48 h à 50°C et la solution ainsi obtenue est filtrée.

Exemple 12

On incorpore 100 g de Codium dans un mélange hydroalcoolique constitué de 180 ml d'eau et 20 ml d'alcool éthylique.

Un broyage est effectué dans les conditions définies a l'exemple 1, puis on réalise une extraction sous légère agitation pendant 48 h à 30°C et on filtre la solution obtenue.

Exemple 13

On plonge 100 g de Fucus vesiculosus, pendant environ 30 min dans une solution hydroalcoolique afin d'inactiver par saponification la lipoxygénase et la catalase et d'éliminer la majorité des lipides.

Après lavage, on effectue une extraction dans 200 ml d'eau pendant 12 h à température ambiante.

L'extrait ainsi obtenu est alors centrifugé à environ 3 000 g puis purifié de la façon suivante :

On ajoute au centrat du sulfate d'ammonium solide en quantité nécessaire pour obtenir une concentration finale de 60 % de la saturation a 5°C. Le précipié est ensuite éliminé par centrifugation (2000-3000 g).

Du sulfate d'ammonium est ajouté au centrat (80 % du seuil de saturation). Une fois mélangé, le précipité est récupéré par centrifugation. Après avoir soigneusement éliminé les traces de surnageant, le précipité est redissous dans une quantité aliquote d'eau tamponnée (pH 8,5).

La solution obtenue est alors appliquée sur une colonne remplie de QAE SEPHAROSE équilibré avec le même tampon. L'extrait est élué. Les fractions contenant plus de 50 % de L'activité maximale sont combinées et concentrées. La fraction réduite est filtrée sur un gel de SEPHACRYL S-300 tamponné à pH 7,8 avec une solution de phosphate de potassium 10 mM. Les fractions contenant plus de 50 % de l'activité maximale sont combinées et concentrées jusqu'à obtenir une concentration en phosphate de 1 mM (pH 7,8).

Le matériel est alors chromatographié sur une colonne d'hydroxyapatite tamponnée à pH 7,8 avec une solution de phosphate 1 mM. Après élution, l'extrait purifié est concentré.

B. Détermination de l'activité antiradicalaire des extraits d'algues, conformes à l'invention

La détermination de l'activité antiradicalaire des extraits d'algues conformes à l'invention a été réalisée en utilisant la technique de Winterbourn. Cette technique est basée sur l'inhibition, par l'enzyme de la réaction de réduction du nitroblue tétrazolium (NBP) provoquée par les radicaux superoxide libres.

Les radicaux libres sont générés par la riboflavine en présence de lumière.

Protocole opératoire

On introduit dans un tube à essais :

. 0,2 ml d'EDTA
. 0,1 ml de NBT
. 0,1 ml de solution enzymatique contenant entre 5 et 10 $\mu$m de protéine
. 2,6 ml de tampon phosphate.

On incube quelques minutes devant une source Lumineuse afin réchauffer aux environs de 30°C le mélange réactionnel.

On rajoute 50 $\mu$l de ribloflavine et on place à nouveau les tubes devant la source lumineuse. Au bout de 12 min, on lit la D0 à 560 nm contre de l'eau distillée.

Un tube témoin dans lequel l'enzyme est remplacée par de l'eau distillée et soumis au même protocole expérimental.

On détermine ainsi le pourcentage d'inhibition (Pi) de la réduction du NBT à l'aide des valeurs de D0 à 560 nm obtenues pour le tube réactionnel et le tube témoin, selon la formule :

$$Pi = (1 - \frac{\text{D0 tube réactionnel}}{\text{D0 tube témoin}}).100$$

La valeur de Pi obtenu doit être proche de 50 % d'inhibition de la réduction du NBT.

Les résultats sont exprimés en unités NBT par gramme d'algues fraîches.

Une unité NBT par gramme est égale à

$$\frac{10^6}{Q_{50}}$$

où $Q_{50}$ est la quantité d'enzymes en µg qui provoque 50 % d'inhibition de la réduction du NBT.

En utilisant la méthode qui vient d'être décrite, on a mesué l'activité antiradicalaire des extraits d'algues obtenus aux exemples 1 à 12.

Les résultats obtenus ont été regroupés au tableau I.

Comme le montre ce tableau, L'activité antiradicalaire est variable selon la nature de l'extrait d'algues.

De très bons résultats sont obtenus avec les extraits de Fucus vesiculosus des exemples 1 et 4.

L'extrait sec obtenu à l'exemple 4 présente une activité antiradicalaire remarquable.

Des résultats très intéressants ont été également obtenus avec des extraits obtenus en soumettant le filtrat de l'exemple 1 à une concentration par ultrafiltration, par osmose inverse et par chromatographie.

TABLEAU I

| Extrait d'algue selon l'exemple | Nature de l'algue utilisée | Activité antiradicalaire Unités NTB par gramme d'algue fraîche |
|---|---|---|
| 1 | Fucus vesiculosus | 11 200 |
| 2 | Aschophyllum nodosum | 1 500 |
| 3 | Pervetia canaliculata | 1 400 |
| 4 | Fucus vesiculosus | 120 000 |
| 5 | Enteromorpha | 1 300 |
| 6 | Palmaria palmata | 260 |
| 7 | Sargassum muticum | 600 |
| 8 | Ceramium rubrum | 400 |
| 9 | Gracilaria verrucosa | 100 |
| 10 | Ulva lactuca | 150 |
| 11 | Laminaria digitata | 100 |
| 12 | Codium | 200 |

On a par ailleurs testé l'effet protecteur des extraits d'algues conformes à l'invention, vis-à-vis de la dégradation du désoxyribose (D.R.) d'origine radicalaire.

D'une façon générale, le test du "désoxyribose" sert à déterminer de façon indirecte la production de métabolites oxygénés radicalaires au sein de système biologique (en mesurant la production de MDA).

Ce test peut être mis en oeuvre de façon relativement aisée, et permet d'obtenir diverses informations quantitatives sur le pouvoir anti-radicalaire d'une molécule in vitro, par sa capacité à inhiber plus ou moins complètement la dégradation du désoxyribose d'origine radicalaire.

On a ainsi déterminé que le taux de protection des extraits d'algues conformes à l'invention était relativement important. Par exemple, l'extrait d'algues selon l'exemple 4 présente un taux de protection de 84, 81, 79, 66 et 38 % pour des taux d'incorporation (volume/volume) de 10, 8, 5, 2 et 1 % respectivement.

Les essais in vitro mentionnés précédemment ont été complétés par un essai in vivo, visant à déterminer

l'activité anti-radicalaire des extraits d'algues conformes à l'invention, lors d'une péroxydation lipidique induite dans des fibroblastes humains en culture par le stress hypoxanthine-xanthine oxydase.

Plus précisément, on a étudié les effets protecteurs des extraits d'algues conformes à l'invention, vis-à-vis des dommages induits par un stress générateur d'ions superoxyde dans le milieu extra-cellulaire.

Cette étude a permis de montrer qu'un extrait d'algues conforme à l'invention, peut, dans certaines conditions, inhiber le processus radicalaire de péroxydation des lipides membranaires de fibroblastes humains en culture.

A titre d'exemple, on peut indiquer ici que l'extrait d'algues selon l'exemple 4, permet d'obtenir un taux d'inhibition de la péroxydation lipidique induite dans les fibroblastes important (de l'ordre de 80 %) pour un taux d'incorporation dans le milieu d'incubation des fibroblastes de 10 % (volume/volume).

Ce taux d'inhibition reste non négligeable (environ 40 %) pour un taux d'incorporation de 2 % (volume/volume).

Les tests in vivo permettent de penser que l'effet anti-radicalaire des extraits d'algues conformes à l'invention, résulte de l'interception des radicaux superoxydes générés dans le milieu extra-cellulaire.

Il a également été montré, à l'aide des tests mentionnés ci-dessus, que certaines substances actives isolées d'extraits d'algues, ou obtenues par synthèse chimique, en particulier les susbtances choisies parmi les fucols, polyfucols, diphlorétols, polyphlorétols, bifuhalols, polyfuhalols, phlorétols, présentaient une activité antiradicalaire.

Ces substances actives pourront être facilement déterminées par l'homme de métier.

Cependant, les essais entrepris par le demandeur ont montré que, parmi les substances isolées d'extraits d'algues et choisies parmi les familles de composés mentionnées précédemment, seuls certains composés de structure chimique déterminée étaient actifs.

D'une façon générale, ces composés actifs sont des fucols, polyfucols, diphlorétols, polyphlorétols, bifuhalols, polyfuhalols, phlorétols (dont la structure chimique est connue) présentant sur chaque noyau aromatique au moins un groupe hydroxyle, et la condition que, lorsqu'un noyau aromatique présente au moins deux groupes hydroxyles, ceux-ci ne soient pas en position méta l'un par rapport à l'autre, sauf dans le cas où trois groupes hydroxyles occupent respectivement trois positions successives sur le noyau aromatique.

Ainsi, dans le cas des fucols, les substances actives sont les composés de formule :

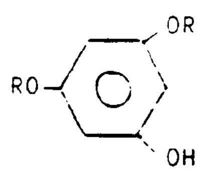

dans laquelle R représente de façon connue, un groupe alkyle ouun sucre,
ainsi que les composés dérivés de ceux-ci par remplacement d'un ou plusieurs atomes d'hydrogéne sur le noyau aromatique par un ou plusieurs atomes d'halogène, ou un ou plusieurs groupes nitro.

Parmi les substances actives isolées d'extraits d'algues conformes à l'invention, les composés les plus intéressants sont les fucohalols et leurs dérivés. Ces composés répondent à la formule générale :

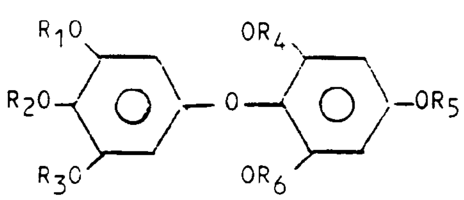

dans laquelle $R_1$, $R_2$, $R_3$, $R_4$, $R_5$, $R_6$ peuvent representer un atome d'hydrogène, un groupe alkyle ou un sucre dans les conditions mentionnées précédemment.

On a notamment constaté que les composés, dans lesquels $R_1$, $R_2$, $R_3$ et éventuellement l'un des groupes $R_4$, $R_5$, $R_5$ représentent un atome d'hydrogène, présentaient une activité antiradicalaire remarquable.

Les extraits d'algues conformes à l'invention ne sont pas toxiques (par voie orale la $DL_{50}$ est supérieure à 10g/kg). Les tests d'irritation cutanée et d'irritation oculaire ont démontré que ces extraits ne sont pas irritants.

D'une façon générale, les extraits d'algues conformes à l'invention peuvent être utilisés pour la préparation de compositions pharmaceutiques radioprotectrices, antisclérodermiques, ou utiles pour le traitement de lésions et brûlures dermiques, de desséchement cutané, d'atonie cutanée ou encore de dermatoses purigineuses.

Dans cette application, on chosira une forme d'administyration permettant un usage externe. L'extrait utilisé peut être pur ou dilué jusqu'à 5 %.

Sous cet aspect, la présente invention vise à couvrir un procédé de traitement thérapeutique du corps humain ou animal, caractérisé en ce qu'il comprend l'administration d'une quantité thérapeutiquement efficace d'au moins un extrait d'algue obtenu par extraction en phase liquide ou d'au moins une substance active isolée d'un tel extrait, ou obtenue par synthèse chimique, en particulier choisie parmi les fucols, polyfucols, diphlorétols, polyphlorétols, bifuhalols, polyfuhalols et phlorétols.

Les compositions cosmétiques incorporant des extraits d'algues conformes à l'invention conviennent particulièrement pour la protection du derme provenant notamment de la protection des acides nucléiques, du collagène de l'acide hyaluronique, des lipides membranaires et des protéines.

On envisage notamment l'élaboration de compositions cosmétiques destinées à la protection contre les réactions phototoxiques, contre l'agression des UV ou au traitement d'érythème actinique.

De telles compositions cosmétiques sont également utiles pour protéger les substances kératiniques vivantes que constituent la peau, le cuir chevelu ou les cheveux.

Les extraits d'algues conformes à l'invention peuvent également être incorporés dans des compositions cosmétiques pour protéger les autres ingrédients contre l'oxydation.

Dans le domaine alimentaire, l'invention permet la réalisation de compositions antioxydantes et efficaces contre la peroxydation des lipides, la dénaturation des enzymes ou la dépolymérisation des polysaccharides. Ces compositions permettent notamment la préservation de la qualité des fruits et des légumes.

Dans le domaine agricole, l'invention permet la réalisation de compositions destinées à améliorer la conservation des graines, fruits, légumes, tubercules et ensilages ou encore à protéger des germinations ou des culutres contre les réactions d'oxydation liées à différents stress (hydrique, froid, osmotiques, carences en éléments nutritifs, pollutions, traitements pesticides).

On donnera ci-après, à titre illustratif des exemples de compositions conformes à l'invention.

Crème traitante :

- Cyclogol NI : 10 %
- Huile minérale "Carnation" : 15 %
- Huile de lanoline : 0,25 %
- Oléate de polypropylène glycol 2000 : 5 %
- Extrait aqueux de Fucus vesiculosus : 10 %
- Parfum : 0,2 %
- Paraben : 0,1 %
- Eau : 59,45 %

Le cyclogol NI de la Société WITCO est un mélange d'alcool cétéarylique et de cétéareth 20.

Schampooing :

- Lauryl sulfate de triéthanolamine : 15 %
- Diéthanolamide de coprah : 2 %
- Extrait aqueux d'Ascophyllum nodosum : 4 %
- Parfum chèvrefeuille : 0,2 %
- Paraben : 0,1 %
- Eau : 78,7 %

Bain moussant :

- Hemi ester sulfosuccinique : 45 %
- Diéthanolamide de coprah : 2,5 %
- Extrait aqueux de Ulva lactuca : 10 %

- EDTA 4 Na : 1 %
- Parfum : 0,3 %
- Paraben : 0,1 %
- Colorant E 131 : 0,01 %
- Eau : 41,1 %

Lotion solaire :

- Giv-Tan F (Givaudan) : 2 %
- OLéate de polypropylène 2000 : 25 %
- Extrait hydroalcoolique de Fucus vesiculosus : 10 %
- Alcool éthyLique : 62,7 %
- Parfum : 0,3 %

Lait de toilette :

- Chlorure de steapyrium : 1 %
- Huile Carnation : 4 %
- Monostéarate de glycérol : 2 %
- Glycérine : 4 %
- Extrait glycolique de Ceramium rubrum : 4 %
- Parfum : 0,3 %
- Eau : 84,7 %

Mousse à raser :

- Acide stéarique : 6,8 %
- Triéthanolamine : 3,7 %
- Propylène glycol 2000 : 0,6 %
- Lauramide : 0,5 %
- Distéarate de polyéthylène glycol 150 : 0,2 %
- Propylène glycol stéarate : 1 %
- Extrait glycolique de Pelvetia canaliculata : 3 %
- Parfum : 0,4 %
- Glycérol : 1 %
- Eau : 82,8 %

Crème radio protectrice :

- Monostéarate de glycérol : 5 %
- Stéarine : 3,6 %
- Huile de paraffine : 7%
- Palmitate de cétyle : 0,4 %
- Alginate de triéthanolamine : 0,8 %
- Extrait glycolique de fucus : 9 %
- Triéthanolamine : 0,4 %
- Parfum : 0,1 %
- Paraben : 0,1 %
- Eau purifiée et déminéralisée : 73,6 %

Cette crème peut être appliquée matin, midi et soir en couches épaisses sur et autour des régions traitées. On fera pénétrer cette crème en massant légèrement.

Crème anti-sénescence :

- Cyclogol NI : 4,5 %
- Cire d'abeille : 13 %
- Extrait glycolique d'ascophyllum : 12 %
- Lanoline : 3 %

- Huile minérale Carnation : 12 %
- Borax : 1,5 %
- Parfum : 0,2 %
- Paraben : 0,1 %
- Eau : 53,7 %.

Fertilisant foliaire Zn :

- Eau : 34,6 %
- Sulfate de Zinc (à 21% Zn) : 23 %
- Potasse : 0,4 %
- Chlorure de Magnésium à 22,5% MgO : 12 %
- Extraits de Bétaines (dosant 10% bétaines) : 20 %
- Extrait aqueux de fucus : 10 %.

Fertilisant foliaire MnCu :

- Sulfate de cuivre (25% Cu) : 6,8 %
- Sulfate de Manganèse (30,8% Mn) : 2,7 %
- Chlorure de Magnésium (22,5% MgO) : 24,4 %
- Extrait de Bétaines (dosant 10% bétaines) : 16,5 %
- Extrait aqueux de Fucus : 49,6 %.

Conservateur de fleurs coupées :

- Extrait aqueux de Fucus : 5 à 50 %
- Sel de cobalt (Sulfate de Cuivre, Chlorure de cobalt, Nitrate de cobalt) : 0,2 mM
- Eau q.s.p.

Conservateur fruits légumes tubercules :

- Extrait aqueux de Fucus : 10 à 95 %
- Acide Ascorbique : 5 - 500 mM
- Acide polyphosphate : 0,1 - 5 %.

Tablette diététique :

- Extrait sec de Fucus : 200 mg
- Cellulose cristalline : 47 mg
- Dextrine : 5 mg
- Lactose : 20 mg
- Carboxyméthylcellulose : 5 mg
- Talc : 3 mg.

Boisson diététique :

- Extrait de Fucus : 1 à 20 %
- Parfum (citron, orange, ...) : 0,5 à 1,0 %
- Acide citrique : 0,5 à 1,0 %
- Eau q.s.p. 1000

Les extraits d'algues conformes à la présente invention présentent de nombreux avantages. Outre une très forte activité antiradicalaire, ces extraits, ou les composés isolés de ces extraits, présentent une stabilité très importante, très Largement supérieure à celle de composés tels que les superoxyde dismutases. Le faible poids moléculaire de ces extraits favorise en outre la pénétration transcutanée. Enfin, ces extraits présentent une inocuité remarquable.

## Revendications

1. Utilisation d'extraits d'algues macroscopiques obtenus par extraction en phase liquide, ou d'au moins une substance active isolée d'un tel extrait ou obtenue par synthèse chimique, en particulier choisie parmi les fucols, polyfucols, diphlorétols, polyphlorétols, bifuhalols, polyfuhalols, phlorétols, pour la préparation de compositions pharmaceutiques, cosmétiques, alimentaires ou à usage agricole à activité antiradicalaire, vis-à-vis du radical superoxyde.

2. Utilisation selon la revendication 1, caractérisée en ce que les algues utilisées sont des algues macroscopiques brunes, vertes ou rouges, des genres Fucus, Pelvetia, Ascophyllum, Himanthalia, Laminaria, Sargassum, Chondrus, Mastocarpus ou Girgatina, Palmaria, Porphyra, Ceramium, Gracilaria, Ulva, Enteromorpha et Codium.

3. Utilisation selon la revendication 1 ou 2 caractérisée en ce que l'algue utilisée est l'une des variétés Fucus vesiculosus, Aschophyllum nodosum, Pelvetia canaliculata, Entermorpha, Palmaria palmata, Sargassum muticum, Ceramium rubum, Gracilaria verrucosa, Ulva lactuca, Laminaria digitata, Codium.

4. Utilisation selon la revendication 1, caractérisée en ce que l'algue utilisée est le Fucus vesiculosus.

5. Utilisation selon l'une quelconque des revendications 1 à 4, caractérisée en ce que l'extrait d'algue utilisé est obtenu par extraction aqueuse à pH contrôlé ou par extraction en solvant polaire, notamment hydroalcoolique ou glycolique, et est éventuellement concentré par séchage sous vide, osmose inverse, chromatographie ou ultrafiltration.

6. Utilisation selon l'une des revendications 1 à 5, caractérisée en ce que la substance active est choisie parmi les fucohalols.

## Patentansprüche

1. Verwendung von durch Extraktion von makroskopischen Algen in flüssiger Phase erhaltenen Extrakten oder mindestens eines aus einem derartigen Extrakt isolierten oder durch chemische Synthese erhaltenen Wirkstoffs. der insbesondere unter den Fucolen, Polyfucolen, Diphloretolen, Polyphloretolen, Bifuhalolen, Polyfuhalolen und Phloretolen ausgewählt ist, zur Herstellung von pharmazeutischen Zusammensetzungen, kosmetischen Zusammensetzungen, Lebensmittelzusammensetzungen oder Zusammensetzungen zum Einsatz in der Landwirtschaft, die gegenüber dem Hyperoxy-Radikal als Radikal-Fänger wirken.

2. Verwendung nach Anspruch 1, dadurch gekennzeichnet, daß die verwendeten Algen makroskopische Braunalgen, Grünalgen oder Rotalgen der Gattungen Fucus, Pelvetia, Ascophyllum, Himanthalia, Laminaria, Sargassum, Chondrus, Mastocarpus oder Girgatina, Palmaria, Porphyra, Ceramium, Gracilaria, Ulva, Enteromorpha und Codium sind.

3. Verwendung nach Anspruch 1 oder 2, dadurch gekennzeichnet, daß die verwendete Alge eine der Algenarten Fucus vesiculosus, Ascophyllum nodosum, Pelvetia canaliculata, Enteromorpha, Palmaria palmata, Sargassum muticum, Ceramium rubrum, Gracilaria verrucosa, Ulva lactuca, Laminaria digitata und Codium ist.

4. Verwendung nach Anspruch 1, dadurch gekennzeichnet, daß die verwendete Alge Fucus vesiculosus ist.

5. Verwendung nach einem der Ansprüche 1 bis 4, dadurch gekennzeichnet, daß der verwendete Algenextrakt durch wäßrige Extraktion bei kontrolliertem pH-Wert oder durch Extraktion in einem polaren Lösungsmittel, insbesondere einem wäßrig-alkoholischen Lösungsmittel oder einem Glycollösungsmittel, erhalten und ggfs. durch Trocknen im Vakuum, Umkehrosmose, Chromatographie oder Ultrafiltration aufkonzentriert ist.

6. Verwendung nach einem der Ansprüche 1 bis 5, dadurch gekennzeichnet, daß der Wirkstoff unter den Fucohalolen ausgewählt ist.

## Claims

1. Utilization of macroscopic algae extracts obtained by extraction in liquid phase, or of at least one active substance isolated from such an extract or obtained by chemical synthesis, selected in particular among the fucols, polyfucols, diphloretols, polyphloretols, bifuhalols, polyfuhalols, phloretols, for the preparation of pharmaceutical, cosmetic, food or agricultural compositions with anti-radical activity, towards the superoxide radical.

2. Utilization according to claim1, characterized in that the algae used are brown, green or red macroscopic algae, of the Fucus, Pelvetia, Ascophyllum, Himanthalia, Laminaria, Sargassum, Chondrus, Mastocarpus or Girgatina, Palmaria, Porphyra, Ceramium, Gracilaria, Ulva, Enteromorpha and Codium species.

3. Utilization according to claim 1 or 2, characterized in that the algae used is one of the Fucus vesiculosus, Aschophyllum nodosum, Pelvetia canaliculata, Entermorpha, Palmaria palmata, Sargassum muticum, Ceranium rubum, Gracilaria verrucosa, Ulva lactuca, Laminaria digitata, Codium species.

4. Utilization according to claim 1, characterized in that the algae used is the Fucus vesiculosus.

5. Utilization according to any one of claims 1 to 4, characterized in that the algae extract used is obtained by aqueous extraction under controlled pH or by extraction in a polar solvent, such as a hydroalcoholic or glycolic solvent, and is optionally concentrated by drying in vacuo, reverse osmosis, chromatography or ultrafiltration.

6. Utilization according to any one of claims 1 to 5, characterized in that the active substance is selected among the fucohalols.